(19) Europäisches Patentamt
European Patent Office
Office européen des brevets

(11) **EP 3 453 343 B1**

(12) **EUROPÄISCHE PATENTSCHRIFT**

(45) Veröffentlichungstag und Bekanntmachung des Hinweises auf die Patenterteilung:
**13.05.2020 Patentblatt 2020/20**

(51) Int Cl.:
**A61B 17/225** *(2006.01)* **A61N 5/10** *(2006.01)*
**A61B 34/20** *(2016.01)* **A61B 90/00** *(2016.01)*

(21) Anmeldenummer: **18192158.6**

(22) Anmeldetag: **03.09.2018**

(54) **MEDIZINISCH-THERAPEUTISCHES SYSTEM**

MEDICALLY THERAPEUTIC SYSTEM

SYSTÈME THÉRAPEUTIQUE MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.09.2017 DE 102017216017**

(43) Veröffentlichungstag der Anmeldung:
**13.03.2019 Patentblatt 2019/11**

(73) Patentinhaber: **Richard Wolf GmbH**
**75438 Knittlingen (DE)**

(72) Erfinder:
• **Burkhardt, Michael**
**75417 Mühlacker (DE)**
• **Kappler, Tanja**
**75394 Oberreichenbach (DE)**
• **Schneider, Peter**
**75433 Maulbronn (DE)**

(74) Vertreter: **Patentanwälte Vollmann Hemmer Lindfeld**
**Partnerschaft mbB**
**Wallstraße 33a**
**23560 Lübeck (DE)**

(56) Entgegenhaltungen:
**EP-A2- 3 047 809 EP-B1- 2 340 781**
**CN-A- 104 815 399 US-A- 5 301 659**
**US-A1- 2009 003 528**

EP 3 453 343 B1

**Beschreibung**

Die vorliegende Offenbarung betrifft ein System zur medizinisch-therapeutischen Behandlung und ein Steuerungsverfahren für solch ein System, insbesondere zur Schallwellenbehandlung wie etwa der extrakorporalen Stoßwellen-Lithotripsie (ESWL). Die vorliegende Offenbarung kann allerdings auch andere Arten von Therapiequellen umfassen, insbesondere zur Strahlentherapie, die in bestimmter Art und Weise auf den Körper eines Patienten ausgerichtet werden müssen.

Schallwellenbehandlungsgeräte werden heutzutage in der Medizin zahlreich angewendet, und zwar nicht nur zur Stoßwellentherapie zum Zwecke der Steinzertrümmerung (Lithotripsie), sondern auch zu zahlreichen anderen Anwendungen, wie beispielsweise zur nichtinvasiven Behandlung des knochennahen Weichteilbereichs oder zur großflächigen Behandlung von beispielsweise Muskelverspannungen, Muskelverhärtungen, Bindegewebsverdichtungen oder auch zur Behandlung von großflächigen Weichteilregionen wie Fett- und Hautgewebe, Cellulitis, chronischen Wunden, Entzündungsherden und dergleichen.

Bei der extrakorporalen Stoßwellen-Lithotripsie (ESWL) können Schallwellen beispielsweise durch mit Hochspannung über Entladekondensatoren und Thyristorschalter konzertiert angeregte Piezoelemente erzeugt werden, die auf einer konkaven Fläche derart verteilt angeordnet sind, dass sich die Schallwellen in einem Fokusbereich zu einem lokal sehr hohen Druck (zum Teil mehr als 100 MPa) überlagern. Der Fokusbereich ist dabei beispielsweise gezielt auf einen im Körper eines Patienten befindlichen Harn- oder Nierenstein gerichtet, so dass der Stein durch den Druck aufgesprengt wird. Die DE 10 2010 055 836 B4 beschreibt bespielweise solch eine piezoelektrische Stoßwellenquelle.

Eine technische Herausforderung stellt bei den bekannten Systemen die Positionierung und Ausrichtung der Therapiequelle bezüglich eines Zielobjekts in einem auf einem Behandlungstisch befindlichen Patienten dar. Die EP 2 340 781 B1 beschreibt diesbezüglich, eine mit Positionsmarkern bestückte Freihand-Ultraschallsonde vorzusehen und deren Position über ein Lokalisierungssystem in Echtzeit zu orten. Das Zielobjekt wird dann basierend auf dieser Ortung so bezüglich einer Therapiequelle positioniert, dass es im Fokuspunkt der Therapiequelle liegt.

Bei dem in der EP 2 340 781 B1 beschriebenen System ist allerdings problematisch, dass sich das Zielobjekt durch natürliche Patientenbewegungen wie etwa Atembewegungen jederzeit leicht verschieben kann. Eine Überwachung und Überprüfung der Position des Zielobjekts sowie eine einfache und schnelle Positionskorrektur sind nicht möglich.

Mit dem System der vorliegenden Offenbarung lässt sich dagegen die Position des Zielobjekts überwachen und überprüfen und ggf. auf sehr einfache Weise eine schnelle Positionskorrektur durchführen.

Gemäß einem ersten Aspekt der vorliegenden Offenbarung wird ein System bereitgestellt zur medizinisch-therapeutischen Behandlung eines Zielobjekts in einem auf einer Behandlungstischfläche befindlichen Patienten, mit, erstens, einer Freihand-Ultraschallsonde, die von einem Anwender des Systems innerhalb eines Ortungsbereichs im Bezugssystem der Behandlungstischfläche manuell räumlich positionier- und ausrichtbar ist, zweitens, einer Therapiequelle, die in mindestens zwei Winkelebenen im Bezugssystem der Behandlungstischfläche auf das Zielobjekt ausrichtbar ist, drittens, einer Therapiequellen-Ultraschallsonde, die an eine festgelegte Position im Bezugssystem der Therapiequelle räumlich positioniert oder positionierbar ist und deren Ausrichtung in dieser Position in den zwei Winkelebenen im Bezugssystem der Therapiequelle festgelegt ist, und viertens, einem Ortungssystem zur Erfassung der räumlichen Position und Ausrichtung der Freihand-Ultraschallsonde innerhalb des Ortungsbereichs im Bezugssystem der Behandlungstischfläche. Das Ortungssystem ist dabei dazu ausgestaltet, sechs Koordinaten für eine vom Anwender gewählte räumliche Position und Ausrichtung der Freihand-Ultraschallsonde mit einer Blickrichtung auf das Zielobjekt zu speichern. Zudem ist die Therapiequelle dazu ausgestaltet, basierend auf den gespeicherten sechs Koordinaten in den mindestens zwei Winkelebenen so im Bezugssystem der Behandlungstischfläche auf das Zielobjekt ausgerichtet zu werden, dass die Therapiequellen-Ultraschallsonde im Bezugssystem der Behandlungstischfläche im Wesentlichen dieselbe Blickrichtung auf das Zielobjekt hat wie die Freihand-Ultraschallsonde zum Zeitpunkt des Speicherns der sechs Koordinaten.

Ein Patient kann im Sinne dieser Offenbarung ein Mensch oder Tier sein. Das Zielobjekt kann beispielsweise ein Nierenstein, ein Gallenstein, ein Harnstein, ein Tumor, eine Metastase oder eine andere zu therapierende pathologische Stelle im oder am Körper eines Patienten sein. Für das Verständnis dieser Offenbarung ist es wichtig, die Begriffe "Position" bzw. "positionieren" und "Ausrichtung" bzw. "ausrichten" zu definieren und voneinander zu unterscheiden. "Position" bzw. "positionieren" bezieht sich hier auf eine räumliche Position bzw. Verschiebung bezüglich eines Bezugssystems. Eine "Position" kann beispielsweise durch einen dreidimensionalen Positionsvektor $(x, y, z)$ in einem mit dem Bezugssystem fest verbundenen rechtshändigen kartesischen Koordinatensystem eindeutig definiert werden. Eine "Ausrichtung" kann beispielsweise durch einen dreidimensionalen Winkellagen-Vektor $(\alpha, \beta, \gamma)$ in einem mit dem Bezugssystem fest verbundenen rechtshändigen kartesischen Koordinatensystem eindeutig definiert werden. Die Position und die Ausrichtung eines Körpers sind in einem Bezugssystem eindeutig durch sechs Koordinaten definiert. Die Koordinaten können bezüglich eines beliebigen Koordinatensystems dargestellt und/oder gespeichert werden.

Ein "Positionieren" ist hier eine Verschiebung bezüglich eines Bezugssystems. Dabei kann ein fest in einem zweiten Bezugssystem angeordneter Körper bezüglich eines ersten Bezugssystems dadurch positioniert werden, dass sich der Körper im ersten Bezugssystem bewegt und/oder sich das erste Bezugssystem gegenüber dem zweiten Bezugssystem bewegt. Analog ist ein "Ausrichten" hier eine Drehung bezüglich eines Bezugssystems. Dabei kann ein fest in einem zweiten Bezugssystem angeordneter Körper bezüglich eines ersten Bezugssystems dadurch ausgerichtet werden, dass sich der Körper im ersten Bezugssystem dreht und/oder sich das erste Bezugssystem gegenüber dem zweiten Bezugssystem dreht. Mit "automatischem" Positionieren und/oder Ausrichten sei hier gemeint, dass das System oder Teile davon unaufgefordert oder auf Befehl des Anwenders, allerdings ohne weiteren Eingriff, Einstellung oder Eingabe seitens des Anwenders, positioniert und/oder ausgerichtet werden können.

Das hierin offenbarte System kann mehrere Bezugssysteme aufweisen, die gegeneinander positionierbar und/oder drehbar sind. Zum einen kann ein Behandlungsraum, in dem das System angeordnet ist, ein erstes Bezugssystem als festes Bezugssystem definieren. Ein Körper, der ortsfest und unbeweglich in diesem ersten Bezugssystem ist, kann dadurch bezüglich eines anderen Bezugssystems positioniert ($\Delta x$, $\Delta y$, $\Delta z$) und/oder ausgerichtet ($\Delta\alpha$, $\Delta\beta$, $\Delta\gamma$) werden, indem dieses andere Bezugssystem bezüglich des ersten Bezugssystems entsprechend positioniert (-$\Delta x$, -$\Delta y$, -$\Delta z$) und/oder ausgerichtet (-$\Delta\alpha$, -$\Delta\beta$, -$\Delta\gamma$) wird.

Die Behandlungstischfläche kann ein zweites Bezugssystem definieren, das vorzugsweise in drei Richtungen (x, y, z) bezüglich des Behandlungsraums positionierbar ist. Die Behandlungstischfläche könnte auch drehbar sein, ist hier aber vorzugsweise bezüglich des Behandlungsraums nicht drehbar. Die Therapiequelle kann ein drittes Bezugssystem definieren, das in drei Richtungen (x, y, z) bezüglich des Behandlungsraums und/oder der Behandlungstischfläche positionierbar und um drei Achsen um Winkel ($\alpha$, $\beta$, $\gamma$) drehbar sein kann. Vorzugsweise ist die Therapiequelle in zwei zueinander orthogonal stehenden Winkelebenen im Behandlungsraum definiert um einen Fokusbereich verschwenkbar, und hat in einer ersten Ausführungsform nur diese zwei Bewegungsfreiheitsgrade ($\alpha$, $\gamma$). Die Therapiequelle kann selbst um eine entlang der Schnittlinie der Winkelebenen verlaufende Achse um einen Winkel $\beta$ drehbar sein, allerdings ist hier vorzugsweise die Therapiequellen-Ultraschallsonde um den Winkel $\beta$ in der Therapiequelle drehbar, wobei die Blickrichtung der Therapiequellen-Ultraschallsonde in Richtung der Drehachse zeigt.

Die vorzugsweise in die Therapiequelle integrierte Therapiequellen-Ultraschallsonde kann also ein viertes Bezugssystem definieren, das in drei Richtungen (x, y, z) bezüglich des Behandlungsraums und/oder der Behandlungstischfläche positionierbar und um drei Achsen um Winkel ($\alpha$, $\beta$, $\gamma$) drehbar sein kann. Die Therapiequellen-Ultraschallsonde kann in der Therapiequelle ortsfest oder positionierbar sein, beispielsweise von einer zurückgezogenen Standby-Position beispielsweise während einer Stoßwellenbehandlung und einer ausgefahrenen Sonographie-Position während einer Ultraschallaufnahme. Solch eine Positionierung findet vorzugsweise entlang der Blickrichtung der Therapiequellen-Ultraschallsonde statt. Die ausgefahrene Sonographie-Position kann in diesem Fall der festgelegten Position im Bezugssystem der Therapiequelle entsprechen.

Das Ortungssystem kann ein fünftes Bezugssystem definieren, das vorzugsweise gegenüber dem Behandlungsraum oder der Behandlungstischfläche bestimmungsgemäß fest positioniert und ausgerichtet ist, wobei sich die Position und/oder Ausrichtung allerdings durch Toleranzen und/oder Schwingungen ändern kann und ggf. überprüft und korrigiert werden kann. Im Bezugssystem des Ortungssystems kann ein fester Ortungsbereich definiert sein, in dem die Freihand-Ultraschallsonde, die Therapiequelle bzw. die Therapiequellen-Ultraschallsonde und vorzugsweise die Behandlungstischfläche sowohl in ihrer Position als auch in ihrer Ausrichtung mittels des Ortungssystems ortbar sind.

Die Freihand-Ultraschallsonde kann damit ein sechstes Bezugssystem definieren, das in drei Richtungen (x, y, z) bezüglich des Behandlungsraums und/oder der Behandlungstischfläche positionierbar und um drei Achsen um Winkel ($\alpha$, $\beta$, $\gamma$) drehbar ist.

Optional kann die Blickrichtung der an der festgelegten Position im Bezugssystem der Therapiequelle positionierten Therapiequellen-Ultraschallsonde entlang der Schnittlinie der zwei Winkelebenen verlaufen oder diese in einem Fokusbereich in einem spitzen Schnittwinkel von höchstens 30° schneiden. Vorzugsweise ist durch die Therapiequelle eine Therapierichtung in Richtung auf einen Fokusbereich definiert, wobei die Therapierichtung entlang der Schnittlinie der zwei Winkelebenen verläuft. Die Blickrichtung und Drehachse der Therapiequellen-Ultraschallsonde ist ebenfalls vorzugsweise in Therapierichtung ausgerichtet oder schneidet diese im Fokusbereich in einem spitzen Schnittwinkel von höchstens 30°. Der Fokusbereich und der Bereich des Zielobjekts überlappen hier vorzugsweise nach erfolgter Positionierung und Ausrichtung im Wesentlichen vollständig.

Optional kann mindestens eine Winkelposition der an der festgelegten Position im Bezugssystem der Therapiequelle positionierten Therapiequellen-Ultraschallsonde mittels des Ortungssystems innerhalb des Ortungsbereichs im Bezugssystem der Behandlungstischfläche erfassbar sein. Vorzugsweise erfasst das Ortungssystem die Verschwenkwinkel $\alpha$, $\gamma$ der Therapiequelle und den Drehwinkel $\beta$ der Therapiequellen-Ultraschallsonde. Dazu weisen die Therapiequelle und/oder die Therapiequellen-Ultraschallsonde Marker auf, deren Positionen vom Ortungssystem innerhalb des Ortungsbereichs erfasst werden können. Alternativ oder zusätzlich können die Winkelstellungen über Sensoren gemessen und an das Ortungssystem übertragen werden.

Optional kann die räumliche Position der Therapiequellen-Ultraschallsonde innerhalb des Ortungsbereichs im Bezugssystem der Behandlungstischfläche mittels des Ortungssystems kontinuierlich erfassbar sein, wobei die Therapiequellen-Ultraschallsonde basierend auf dieser kontinuierlichen Erfassung im Bezugssystem der Behandlungstischfläche mittels räumlichen Positionierens der Behandlungstischfläche und/oder der Therapiequelle in drei Achsen positionierbar ist.

Optional kann die Therapiequelle einen Stoßwellengenerator eines extrakorporalen Stoßwellen-Lithotripsie-Systems (ESWL) aufweisen. Der Stoßwellengenerator kann eine Form einer Kalotte aufweisen und dabei im Wesentlichen rotationssymmetrisch bezüglich der Therapieachse aufgebaut sein, die durch den Fokusbereich verläuft. Der Fokusbereich kann im Bezugssystem der Therapiequelle fest positioniert sein. Alternativ dazu kann, beispielsweise durch entsprechende Befüllung der Kalotte mit einem flüssigen Schallwellenträger, der Abstand des Fokusbereichs von der Kalotte (ähnlich einer einstellbaren Brennweite einer verformbaren Linse) in gewissen Grenzen eingestellt werden.

Optional kann das Ortungssystem dazu ausgestaltet sein, die sechs Koordinaten auf Befehl eines Anwenders zu speichern. Ein Anwender kann mit der Freihand-Ultraschallsonde einfach und schnell eine gute Blickrichtung auf das Zielobjekt suchen und dem Ortungssystem befehlen, z.B. per Knopfdruck oder Sprachbefehl, diese Position und Ausrichtung der Freihand-Ultraschallsonde im Bezugssystem der Behandlungstischfläche zu speichern. Danach kann die Freihand-Ultraschallsonde weggelegt werden und die Therapiequelle samt Therapiequellen-Ultraschallsonde so im Bezugssystem der Behandlungstischfläche positioniert und ausgerichtet werden, dass die Therapiequellen-Ultraschallsonde im Wesentlichen dieselbe Blickrichtung auf das Zielobjekt hat wie die Freihand-Ultraschallsonde zum Zeitpunkt des Speicherns der sechs Positions- und Ausrichtungskoordinaten.

Die Freihand-Ultraschallsonde ist vorzugsweise ergonomisch ausgestaltet, um dem Anwender eine einfache Handhabung und ausreichend genaue Bildgebung zu bieten, das Zielobjekt und eine gute Blickrichtung darauf schnell und ohne tiefere Kenntnisse von der Therapiequelle zu finden. Die Therapiequellen-Ultraschallsonde ist dagegen vorzugweise mit höherer Präzision und feinerer Auflösung auf einem engeren Sichtbereich ausgestattet, um eine Feinpositionierung und ggf. ein Nachfahren ("Tracking") des Fokusbereichs auf den Bereich des Zielobjekts bei natürlichen Bewegungen des Patienten durchführen zu können. Ein Tracking kann während der Behandlung und/oder zwischen Behandlungszyklen durchgeführt werden.

Optional kann die Therapiequelle einen definierten Fokusbereich im Bezugssystem der Therapiequelle aufweisen und die Therapiequelle im Bezugssystem der Behandlungstischfläche, basierend auf einer kontinuierlichen Erfassung der räumlichen Position der Therapiequellen-Ultraschallsonde innerhalb des Ortungsbereichs im Bezugssystem der Behandlungstischfläche und basierend auf einem Ultraschallbild der Therapiequellen-Ultraschallsonde vom Zielobjekt, mittels räumlichen Positionierens der Behandlungstischfläche und/oder der Therapiequelle in drei Achsen derart positionierbar sein, dass der Fokusbereich dem Zielobjekt nachführbar ist. Alternativ oder zusätzlich kann der Fokusbereich in Blickrichtung der Therapiequellen-Ultraschallsonde durch entsprechende Befüllung einer Kalotte der Therapiequelle mit einem flüssigen Schallwellenträger ähnlich einer einstellbaren Brennweite einer verformbaren Linse verschoben bzw. dem Bereich des Zielobjekts nachgeführt werden.

Gemäß einem zweiten Aspekt der vorliegenden Offenbarung wird ein Steuerungsverfahren für ein medizinisch-therapeutisches System bereitgestellt mit den Schritten:

- Erfassen der räumlichen Position und Ausrichtung einer Freihand-Ultraschallsonde im Bezugssystem einer Behandlungstischfläche mittels eines Ortungssystems,

- Speichern von sechs die räumliche Position und Ausrichtung der Freihand-Ultraschallsonde bestimmenden Koordinaten, und

- Ausrichten einer Therapiequelle basierend auf den gespeicherten sechs Koordinaten in mindestens zwei Winkelebenen im Bezugssystem der Behandlungstischfläche, sodass eine an eine festgelegte Position im Bezugssystem der Therapiequelle positionierte Therapiequellen-Ultraschallsonde im Wesentlichen dieselbe Blickrichtung hat wie die Freihand-Ultraschallsonde zum Zeitpunkt des Speicherns der sechs Koordinaten.

Das hierin offenbarte Steuerungsverfahren erlaubt es einem Anwender ohne tiefere Kenntnis von der Therapiequelle, mit einer Freihand-Ultraschallsonde schnell und einfach ein Zielobjekt sowie eine gute Blickrichtung darauf zu finden und die Ausrichtung der Therapiequelle automatisch durchführen zu lassen, sodass sich die Position des Zielobjekts vor oder während eines Behandlungszyklus überwachen bzw. überprüfen lässt und ggf. ein Fokusbereich der Therapiequelle dem Zielobjekt automatisch nachführen ("tracken") lässt.

Optional kann das Steuerungsverfahren ein Schritt des Drehens der an der festgelegten Position im Bezugssystem der Therapiequelle positionierten Therapiequellen-Ultraschallsonde um eine in ihrer Blickrichtung verlaufende Drehachse im Bezugssystem der Therapiequelle aufweisen. Somit kann vor oder während eines Behandlungszyklus die Blickebene der Therapiequellen-Ultraschallsonde gedreht werden, um in verschiedenen Blickebenen das Zielobjekt tracken zu können, ohne die Therapiequelle selbst drehen zu müssen.

Optional kann die Blickrichtung der an der festgelegten Position im Bezugssystem der Therapiequelle positionierten Therapiequellen-Ultraschallsonde entlang der Schnittlinie der zwei Winkelebenen verlaufen oder diese in einem Fokusbereich in einem spitzen Schnittwinkel von höchstens 30° schneiden. Vorzugsweise ist die Therapiequellen-Ultraschallsonde zentral in der Therapiequelle angeordnet, sodass eine Therapieachse, auf der ein Fokusbereich der Therapiequelle liegt, mit der Blickrichtung der Therapiequellen-Ultraschallsonde im Wesentlichen übereinstimmt.

Optional kann das Steuerungsverfahren einen Schritt des Erfassens mindestens einer Winkelposition der an der festgelegten Position im Bezugssystem der Therapiequelle positionierten Therapiequellen-Ultraschallsonde mittels des Ortungssystems innerhalb des Ortungsbereichs im Bezugssystem der Behandlungstischfläche aufweisen. Damit kann während oder vor einem Behandlungszyklus die mindestens eine, vorzugsweise allerdings zwei, Winkelposition(en) der Therapiequelle bzw. der Therapiequellen-Ultraschallsonde mittels des Ortungssystems erfasst und entsprechend nachgeführt werden, um den Fokusbereich im Bereich des Zielobjekts zu halten.

Optional kann das Steuerungsverfahren einen Schritt des kontinuierlichen Erfassens der räumlichen Position der Therapiequellen-Ultraschallsonde innerhalb des Ortungsbereichs im Bezugssystem der Behandlungstischfläche mittels des Ortungssystems, und einen Schritt des Positionierens der Therapiequellen-Ultraschallsonde im Bezugssystem der Behandlungstischfläche mittels räumlichen Positionierens der Behandlungstischfläche und/oder der Therapiequelle in drei Achsen basierend auf der kontinuierlichen Erfassung aufweisen. Damit kann also durch Positionieren der Behandlungstischfläche und/oder der Therapiequelle der Fokusbereich der Therapiequelle auf dem Zielobjekt gehalten und getrackt werden und die Therapie- bzw. Blickrichtung über die Ausrichtung der in die Therapiequelle integrierten Therapiequellen-Ultraschallsonde basierend auf der zuvor gewählten Ausrichtung der Freihand-Ultraschallsonde eingestellt werden.

Optional kann der Schritt des Speicherns der sechs Koordinaten auf Befehl eines Anwenders erfolgen. Der Anwender kann also beispielsweise per Knopfdruck oder Sprachbefehl die Position und Ausrichtung der Freihand-Ultraschallsonde bestimmen, die eine gute Blickrichtung auf das Zielobjekt bieten. Das Ausrichten und Positionieren der Therapiequelle bzw. der Therapiequellen-Ultraschallsonde kann dann vorzugsweise automatisch erfolgen, um mit der vorzugsweise präziseren, höher aufgelösten und stärker auf das Zielobjekt gezoomten Therapiequellen-Ultraschallsonde aus der mit der Freihand-Ultraschallsonde bestimmten Blickrichtung das Zielobjekt zu tracken und es auch vorzugsweise aus dieser Therapierichtung zu behandeln. Alternativ kann das Ausrichten und Positionieren der Therapiequelle bzw. der Therapiequellen-Ultraschallsonde manuell erfolgen, wobei dem Anwender Informationen für das Ausrichten und Positionieren gegeben werden können, wie etwa Koordinaten relativ zu Zielkoordinaten oder Richtungssteuerhinweise.

Optional kann das Steuerungsverfahren einen Schritt des Positionierens eines im Bezugssystem der Therapiequelle definierten Fokusbereichs, basierend auf einer kontinuierlichen Erfassung der räumlichen Position der Therapiequellen-Ultraschallsonde innerhalb des Ortungsbereichs im Bezugssystem der Behandlungstischfläche und basierend auf einem Ultraschallbild der Therapiequellen-Ultraschallsonde von einem Zielobjekt, mittels räumlichen Positionierens der Behandlungstischfläche und/oder der Therapiequelle in drei Achsen derart aufweisen, dass der Fokusbereich dem Zielobjekt nachgeführt wird. Damit wird ein Zielobjekt automatisch vor oder während einer Behandlung "getrackt".

Die Offenbarung ist nachfolgend anhand von in den Zeichnungen dargestellten Ausführungsbeispielen näher erläutert. Es zeigen:

Fig. 1 eine erste beispielhafte Ausführungsform des hierein offenbarten Systems;

Fig. 2 eine zweite beispielhafte Ausführungsform des hierein offenbarten Systems;

Fig. 3a,b schematisch zwei beispielhafte Positionen des Bezugssystems der Therapiequellen-Ultraschallsonde des hierein offenbarten Systems jeweils vor und nach einer Ausrichtung und Positionierung;

Fig. 4 schematisch ein beispielhaftes Ultraschallbild der Freihand-Ultraschallsonde des hierein offenbarten Systems auf ein Zielobjekt; und

Fig. 5 eine beispielhafte Ausführungsform des hierein offenbarten Steuerungsverfahrens.

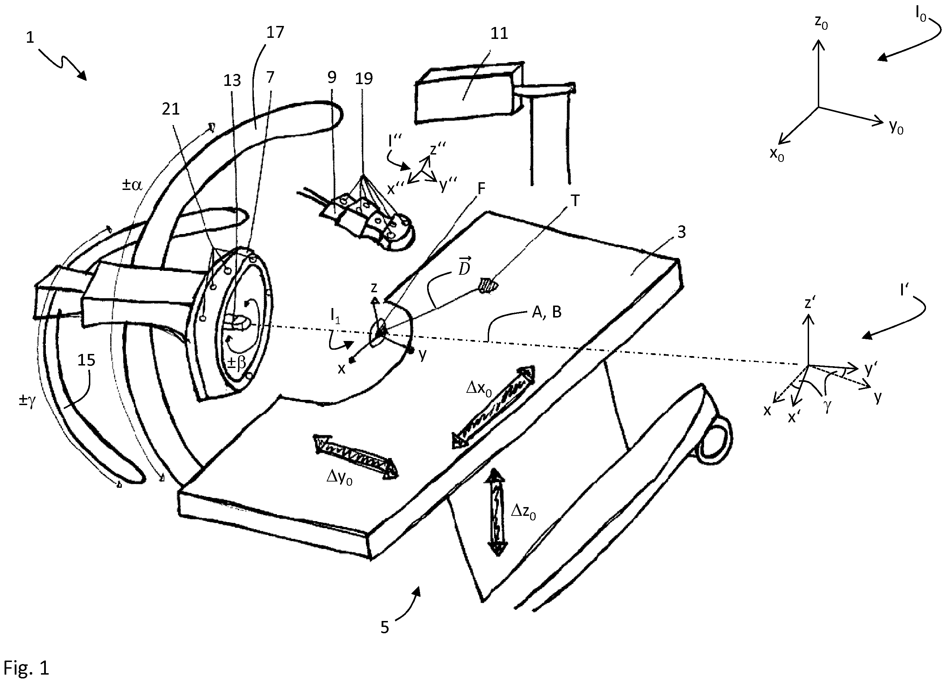

In Fig. 1 ist ein System 1 gezeigt zur medizinisch-therapeutischen Behandlung eines Zielobjekts T in einem auf einer Behandlungstischfläche 3 befindlichen Patienten. Das Zielobjekt T kann beispielsweise ein Nierenstein in einem Patienten sein, der auf der Behandlungstischfläche 3 liegt, aber hier nicht gezeigt ist. Das System 1 ist in einem Behandlungsraum angeordnet, der ein festes Bezugssystem $I_0$ als Bezugssystem definiert, das mit einem rechtshändigen kartesischen Koordinatensystem mit den Achsen $x_0$, $y_0$ und $z_0$ gekennzeichnet ist. Das System 1 weist hier zunächst einen im Behandlungsraum verschiebbaren oder verfahrbaren Behandlungstisch 5 mit der im Wesentlichen horizontalen Behandlungstischfläche 3 auf. Außerdem weist das System 1 eine Therapiequelle 7 in Form eines Stoßwellengenerators als Teil eines extrakorporalen Stoßwellen-Lithotripsie-Systems (ESWL) auf. Zudem umfasst das System 1 eine Freihand-Ultraschallsonde 9 und ein Ortungssystem 11. Schließlich weist das System 1 eine zentral in die Therapiequelle 7 integrierte Therapiequellen-Ultraschallsonde 13 auf.

Die Position des Behandlungstisches 5 im Bezugssystem $l_0$ wird vor einer Behandlung festgelegt und bleibt dabei unverändert, sodass das Bezugssystem des Behandlungstisches 5 mit dem Bezugssystem $l_0$ übereinstimmt. Die Behandlungstischfläche 3 ist hingegen in bestimmten Grenzen um $\Delta x_0$, $\Delta y_0$ und $\Delta z_0$ positionierbar in den Achsen $x_0$, $y_0$ und $z_0$ und definiert damit ein gegenüber dem Bezugssystem $l_0$ positionierbares Bezugssystem $l_1$ der Behandlungstischfläche 3. Das Zielobjekt T ist über die ruhiggestellte Patientenposition auf der Behandlungstischfläche 3 im Bezugssystem $l_1$ im Wesentlichen festgelegt. Allerdings kann es durch natürliche Patientenbewegungen, wie etwa Atmen, zu leichten Verschiebungen des Zielobjekts T im Bezugssystem $l_1$ der Behandlungstischfläche 3 kommen.

Die Therapiequelle 7 ist im Bezugssystem $l_0$ des Behandlungsraums und im Bezugssystem $l_1$ der Behandlungstischfläche 3 um die vertikale Achse $z_0$ bzw. z in gewissen Grenzen um einen Winkel $\pm\gamma$ in einer ersten Winkelebene xy und um die horizontale Achse $x_0$ bzw. x in gewissen Grenzen um einen Winkel $\pm\alpha$ in einer zweiten Winkelebene zy verschwenkbar angeordnet. Dazu ist die Therapiequelle 7 über zwei zueinander orthogonal ausgerichtete kreisabschnittsförmige Führungsbögen 15, 17 aufgehängt. Mittels Motoren und Winkelgeber (nicht gezeigt) können die Winkel $\alpha$ und $\gamma$ gesteuert eingestellt werden. Die Führungsbögen 15, 17 können am Behandlungstisch 5 oder separat davon im Behandlungsraum befestigt sein. Die Therapiequelle 7 definiert damit ein gegenüber dem Behandlungsraum ausrichtbares Bezugssystem l' mit den Achsen x', y' und z', das in Fig. 1 um einen Winkel $\gamma$ gegenüber dem Bezugssystem $l_1$ der Behandlungstischfläche 3 um die vertikale z-Achse gedreht ist.

Alternativ zu den zwei Führungsbögen 15, 17 kann auch nur ein Führungsbogen 17 beispielsweise zur Verschwenkung um die horizontale Achse $x_0$ bzw. x um den Winkel $\pm\alpha$ vorgesehen sein, wobei der Führungsbogen 17 selbst um die horizontale Achse $y_0$ bzw. y um den Winkel $\pm\beta$ drehbar gelagert ist. Eine Verschwenkung um die vertikale Achse $z_0$ bzw. z um einen Winkel $\pm\gamma$ kann dann durch eine Kombination aus einer Drehung des Führungsbogens 17 um $\pm\beta$ und einer Verschwenkung der Therapiequelle entlang des Führungsbogens 17 um $\pm\alpha$ erzielt werden.

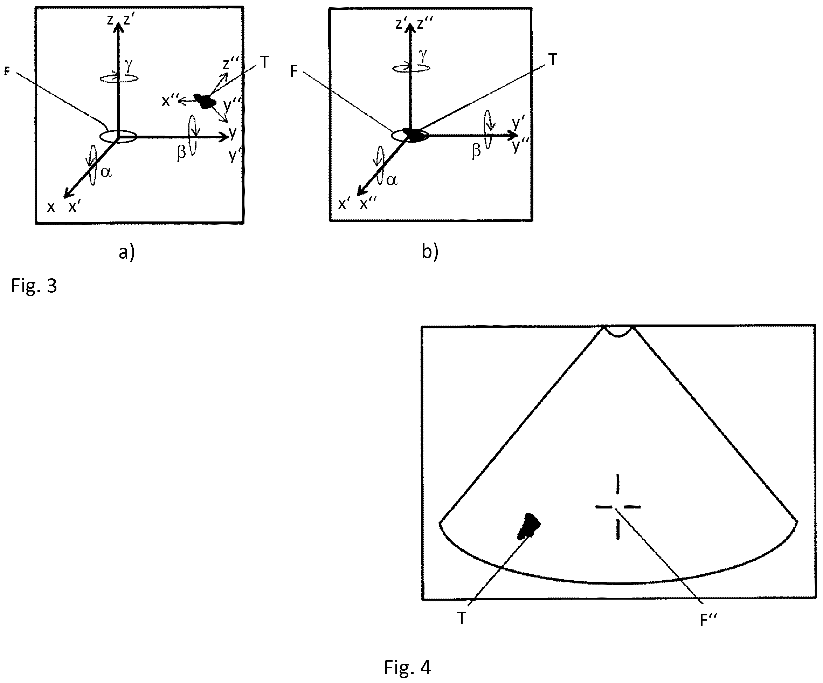

Die Therapiequelle 7 weist eine im Wesentlichen bezüglich einer Therapieachse A, die hier in y'-Richtung verläuft, rotationssymmetrische Kalotte eines Stoßwellengenerators auf. Mittels einer Anordnung von einer Vielzahl von Piezoelementen auf der konkaven Innenfläche ist die Therapiequelle 7 dazu ausgestaltet, Schallwellenstöße in Richtung der Therapieachse A auszusenden und in einem auf der Therapieachse A liegenden Fokusbereich F zu fokussieren. Die Überlagerung der Schallwellenstöße im Fokusbereich F führt im Fokusbereich F zu derart hohen Schallwellenamplituden, dass sich damit das Zielobjekt T, beispielsweise in Form eines Nierensteins, zertrümmern lässt, sofern sich das Zielobjekt T im Fokusbereich F befindet. In Fig. 1 liegt das Zielobjekt T um einen Distanzvektor $\vec{D} = \begin{pmatrix} \Delta x_0 \\ \Delta y_0 \\ \Delta z_0 \end{pmatrix}$ versetzt zum Fokusbereich F (siehe auch Fig. 3a). Durch Positionieren der Behandlungstischfläche 3 um $\Delta x_0$, $\Delta y_0$ und $\Delta z_0$ kann das Zielobjekt T in den Fokusbereich F gebracht werden (siehe Fig. 3b). Im Bezugssystem $l_1$ der Behandlungstischfläche 3 wird dadurch der Fokusbereich F um $-\Delta x$, $-\Delta y$ und $-\Delta z$ auf das Zielobjekt T positioniert, ohne die Koordinaten $x_0$, $y_0$ und $z_0$ des Fokusbereichs F im Behandlungsraum zu ändern.

Die Führungsbögen 15, 17 sind hier derart aufgebaut, dass sich der Fokusbereich F bei Verschwenken der Therapiequelle 7 um die Winkel $\alpha$ und $\gamma$ im Wesentlichen nicht im Bezugssystem $l_0$ des Behandlungsraums verschiebt, d.h. die beiden Schwenkachsen $z_0$ und $x_0$ verlaufen durch den Fokusbereich F. In Richtung der Therapieachse A kann der Fokusbereich F ggf. durch eine einstellbare Befüllung der Kalotte mit einem flüssigen Schallwellenträger in gewissen Grenzen verschoben werden, ähnlich einer einstellbaren Brennweite einer verformbaren Linse. Vorzugsweise ist die Position des Fokusbereichs F im Bezugssystem $l_0$ des Behandlungsraums jedoch festgelegt, allerdings nicht die Therapieachse A.

Die Therapieachse A stimmt hier mit einer Blickrichtung B der zentral in die Therapiequelle 7 integrierten Therapiequellen-Ultraschallsonde 13 überein. Durch die festgelegte Position im Bezugssystem l' der Therapiequelle weist die Blickrichtung B immer in y'-Richtung. Die Therapiequellen-Ultraschallsonde 13 ist in der Therapiequelle 7 allerdings drehbar um einen Winkel $\beta$ bezüglich der Therapieachse A bzw. y'. Die Therapiequellen-Ultraschallsonde 13 ist also in der Lage, in den Ebenen x'y' und z'y' sowie allen anderen Ebenen, in denen die y'-Achse liegt, zu sonographieren.

Die Freihand-Ultraschallsonde 9 ist völlig frei im Behandlungsraum positionier- und ausrichtbar. Damit definiert die Freihand-Ultraschallsonde 9 ein weiteres Bezugssystem l" mit den Achsen x", y" und z". Mit Hilfe der Freihand-Ultraschallsonde 9 kann ein Anwender auf einfache und ihm geläufige Weise das Zielobjekt T im Patienten sonographisch suchen und einen guten Blickwinkel auf das Zielobjekt T bestimmen, aus dem das Zielobjekt T anschließend mit der Therapiequelle 7 behandelt werden soll. Die Blickrichtung der Freihand-Ultraschallsonde 9 verläuft hier in y"-Richtung. Das Ortungssystem 11 ist nun in der Lage, zu bestimmen, wie die Therapiequelle 7 verschwenkt werden muss, damit die Therapieachse A mit der mit dem guten Blickwinkel mittels der Freihand-Ultraschallsonde 9 bestimmten y"-Richtung übereinstimmt.

Dazu ist das Ortungssystem 11 vorzugsweise mit mindestens zwei voneinander beabstandeten Kameras bestückt, die optisch die Position und Ausrichtung der Freihand-Ultraschallsonde 9 orten können. Das Ortungssystem 11 hat einen begrenzten Ortungsbereich, der sich vorzugsweise so weit über und um den Behandlungstisch 5 erstreckt, dass die Freihand-Ultraschallsonde 9 bei Körperkontakt mit einem auf der Behandlungstischfläche 3 befindlichen Patienten ortbar ist. Zur Ortung der Position und Ausrichtung der Freihand-Ultraschallsonde 9 können auf dieser mindestens drei, vorzugsweise vier oder mehr, Marker 19 angeordnet sein, sodass durch die zwei Kamerabilder von den Markern 19 eindeutig sechs Koordinaten $\begin{pmatrix} x \\ y \\ z \end{pmatrix}$ und $\begin{pmatrix} \alpha \\ \beta \\ \gamma \end{pmatrix}$ bestimmbar sind. Das Ortungssystem 11 kann mit der Behandlungstischfläche 3 fest gekoppelt sein, sodass es fest im Bezugssystem $I_1$ ist. Alternativ dazu kann es auch separat davon im Behandlungsraum angeordnet sein, wobei in diesem Fall die Behandlungstischfläche 3 ebenfalls mindestens drei, vorzugsweise vier oder mehr, Marker (nicht gezeigt) aufweisen kann, um die Position des Bezugssystems $I_1$ der Behandlungstischfläche 3 zu bestimmen und aus den Koordinaten $\begin{pmatrix} x_0 \\ y_0 \\ z_0 \end{pmatrix}$ und $\begin{pmatrix} \alpha_0 \\ \beta_0 \\ \gamma_0 \end{pmatrix}$ auf die Koordinaten $\begin{pmatrix} x \\ y \\ z \end{pmatrix}$ und $\begin{pmatrix} \alpha \\ \beta \\ \gamma \end{pmatrix}$ schließen zu können. Die Ortung von Markern auf der Behandlungstischfläche 3 mittels des Ortungssystems 11 kann selbst dann sinnvoll sein, wenn das Ortungssystem 11 eigentlich fest mit der Behandlungstischfläche 3 fest gekoppelt sein soll. Schwingungen und Toleranzen können nämlich dazu führen, dass die tatsächliche Position und Ausrichtung des Ortungssystem 11 bezüglich der Behandlungstischfläche 3 nicht der Sollposition und Sollausrichtung entsprechen. Mittels der Ortung der Behandlungstischfläche 3 können die tatsächliche Position und Ausrichtung des Ortungssystem 11 bezüglich der Behandlungstischfläche 3 überprüft und entsprechend kalibriert werden.

Das Ortungssystem 11 kann also die Koordinaten $\begin{pmatrix} x \\ y \\ z \end{pmatrix}$ und $\begin{pmatrix} \alpha \\ \beta \\ \gamma \end{pmatrix}$ der Freihand-Ultraschallsonde 9 im Bezugssystem $I_1$ der Behandlungstischfläche 3 kontinuierlich erfassen. Der Anwender kann per Knopfdruck oder Sprachbefehl dem Ortungssystem 11 befehlen, die sechs Koordinaten $\begin{pmatrix} x \\ y \\ z \end{pmatrix}$ und $\begin{pmatrix} \alpha \\ \beta \\ \gamma \end{pmatrix}$ der momentanen Position und Ausrichtung zu speichern, wenn die Freihand-Ultraschallsonde 9 aus gutem Blickwinkel auf das Zielobjekt T gerichtet ist. Die Entscheidung, welcher Blickwinkel hierbei gut ist, ist dem Anwender in Grenzen frei überlassen. Die Grenzen hierfür bilden lediglich die Maximalwinkel $\pm\gamma_{max}$ und $\pm\alpha_{max}$ der Verschwenkbarkeit der Therapiequelle 7, die einen Raumwinkel aufspannen, in dem die Therapieachse A liegen kann.

Um die Therapieachse A mit der mittels der Freihand-Ultraschallsonde 9 bestimmten y"-Richtung in Übereinstimmung bringen zu können, kann das Ortungssystem 11 auch die Winkellagen $\gamma$ und $\alpha$ der Therapiequelle 7 kontinuierlich erfassen. Dazu weist die Therapiequelle 7 ebenfalls drei, vorzugsweise vier oder mehr, Marker 21 auf, die im Ortungsbereich des Ortungssystems 11 von diesem ortbar sind. Die Marker 21 der Therapiequelle 7 können sich durch Form, Farbe, Helligkeit und/oder Struktur von den Marker 19 der Freihand-Ultraschallsonde 9 unterscheiden, damit das Ortungssystem 11 diese eindeutig der Therapiequelle 7 bzw. der Freihand-Ultraschallsonde 9 zuordnen kann. Damit sind auch die Winkellagen $\gamma$ und $\alpha$ der integrierten Therapiequellen-Ultraschallsonde 13 im Bezugssystem $I_1$ der Behandlungstischfläche 3 kontinuierlich erfassbar. Die Winkellage $\beta$ der Therapiequellen-Ultraschallsonde 13 in der Therapiequelle 7 kann hier ebenfalls über Marker an der Therapiequellen-Ultraschallsonde 13 oder durch Winkelmesser in der Therapiequelle 7 bestimmt werden. Allerdings ist dies nicht zwingend notwendig, um die Therapieachse A auf die gespeicherte Blickrichtung der Freihand-Ultraschallsonde 9 in y"-Richtung zu bringen. Dazu genügt die Erfassung der Winkellagen $\gamma$ und $\alpha$. Auch die Position der Therapiequelle 7 kann bezüglich der Behandlungstischfläche 3 im Bezugssystem $I_1$ mittels des Ortungssystems 11 erfassbar sein, wenngleich diese über eine feste nominelle Position der Führungsbögen 15, 17 bezüglich des Behandlungstisches 5 bestimmbar sein kann. Auch hier können Schwingungen und Toleranzen dazu führen, dass es sinnvoll ist, die tatsächliche Position der Therapiequelle 7 mittels des Ortungssystems 11 zu überprüfen und ggf. entsprechend zu kalibrieren. Damit ist auch die Position des Fokusbereichs F, der eine fest definierte Lage zur Therapiequelle 7 hat, im Bezugssystem $I_1$ der Behandlungstischfläche 3 bekannt.

Das Ortungssystem 11 hat nun genug Informationen, um die Winkellagenunterschiede $\Delta\gamma$ und $\Delta\alpha$ zwischen dem Bezugssystem I' und dem Bezugssystem I" zu bestimmen, um welche die Therapiequelle 7 verschwenkt werden muss, damit die Therapieachse A bzw. die Blickrichtung B der Therapiequellen-Ultraschallsonde 13 parallel zur y"-Richtung gemäß der gespeicherten Koordinaten der Freihand-Ultraschallsonde 9 verläuft.

Nun muss nur noch der Distanzvektor $\vec{D} = \begin{pmatrix} \Delta x_0 \\ \Delta y_0 \\ \Delta z_0 \end{pmatrix}$ bestimmt werden, um den der Fokusbereich F versetzt zum Zielobjekt T liegt, um die Therapieachse A bzw. die Blickrichtung B auf die y"-Richtung gemäß der gespeicherten Koordinaten der Freihand-Ultraschallsonde 9 zu verschieben und den Fokusbereich F auf das Zielobjekt T zu legen. Im Ultraschallbild der Freihand-Ultraschallsonde 9 kann wie in Fig. 4 gezeigt eine Markierung des Soll-Fokusbereichs F'' an einer festgelegten Position im Bezugssystem I'' markiert sein. Der Soll-Fokusbereich F'' ist also im Ultraschallbild der Freihand-Ultraschallsonde 9 immer, vorzugsweise mittig, an der gleichen Stelle. Speichert der Anwender die Position und Ausrichtung der Freihand-Ultraschallsonde 9, wenn das Zielobjekt T im Soll-Fokusbereich F'' liegt, so ist über die feste Beziehung zwischen dem Soll-Fokusbereich F'' und der Freihand-Ultraschallsonde 9 die Position des Zielobjekts T im Bezugssystem $I_1$ der Behandlungstischfläche 3 bekannt. Im Ultraschallbild der Therapiequellen-Ultraschallsonde 13 kann analog eine Markierung des tatsächlichen Fokusbereichs F an einer festgelegten Position im Bezugssystem I' der Therapiequelle markiert sein. Für den Fall, dass die "Brennweite" der Therapiequelle 7 einstellbar ist, ist die Markierung des tatsächlichen Fokusbereichs F vorzugsweise samt Ultraschallbildausschnitt der Therapiequellen-Ultraschallsonde 13 sowie die Markierung des Soll-Fokusbereichs F'' vorzugsweise samt Ultraschallbildausschnitt der Freihand-Ultraschallsonde 9 entsprechend einstellbar bzw. direkt daran gekoppelt.

Da also im Bezugssystem $I_1$ der Behandlungstischfläche 3 sowohl die Position des Zielobjekts T als auch die Position des Fokusbereichs F der Therapiequelle 7 bekannt sind, ist auch der Distanzvektor

$$\vec{D} = \begin{pmatrix} \Delta x_0 \\ \Delta y_0 \\ \Delta z_0 \end{pmatrix}$$ bekannt, um den die Behandlungstischfläche 3 im Behandlungsraum verfahren werden muss, um das Zielobjekt T in den Fokusbereich F zu bringen. Fig. 3a zeigt diesbezüglich schematisch den Ausgangspunkt und Fig. 3b den Endpunkt nach erfolgter Positionierung, um das Zielobjekt T in den Fokusbereich F zu bringen. Die Therapieachse A der Therapiequelle 7 bzw. die Blickrichtung B der Therapiequellen-Ultraschallsonde 13 liegt dann im Bezugssystem $I_1$ der Behandlungstischfläche 3 genau auf der y''-Achse wie sie zu dem Zeitpunkt positioniert und ausgerichtet war als die sechs Koordinaten der Freihand-Ultraschallsonde 9 gespeichert wurden. Auch der Drehwinkel β der Therapiequellen-Ultraschallsonde 13 kann so eingestellt werden, dass das Ultraschallbild der Therapiequellen-Ultraschallsonde 13 exakt mit der Sonographieebene übereinstimmt, welche die Freihand-Ultraschallsonde 9 zum Zeitpunkt des Speicherns hatte.

Der Anwender kann also von der Freihand-Ultraschallsonde 9 mit vorzugsweise größerem Ultraschallbildausschnitt und schnellerer Bildverarbeitung per Befehl auf die Therapiequellen-Ultraschallsonde 13 mit vorzugsweise herangezoomtem Ultraschallbildausschnitt und höherer Auflösung umschalten, indem er eine gewünschte Position und Ausrichtung der Freihand-Ultraschallsonde 9 speichert und damit eine automatische oder durch weiteren Befehl auslösbare entsprechende Positionierung der Behandlungstischfläche 3 und Ausrichtung der Therapiequelle 7 auslöst. Sobald die Therapiequelle 7 entsprechend ausgerichtet und die Behandlungstischfläche 3 positioniert ist, steht dem Anwender das Ultraschallbild der hochpräzisen Therapiequellen-Ultraschallsonde 13 genau auf das Zielobjekt T aus derselben Blickrichtung zur Verfügung, vorzugsweise sogar in derselben Sonographieebene. Außerdem ist die Therapieachse A bereits wie gewünscht ausgerichtet. Nun kann mittels des Ultraschallbilds der hochpräzisen Therapiequellen-Ultraschallsonde 13 die Position der Behandlungstischfläche 3 ggf. so nachgeführt werden, dass das Zielobjekt T bei natürlichen Bewegungen des Patienten nicht aus dem Fokusbereich F wandert. Das Zielobjekt T kann dazu beispielsweise im Ultraschallbild der hochpräzisen Therapiequellen-Ultraschallsonde 13 durch den Anwender und/oder einen Zielobjekt-Erkennungsalgorithmus markiert werden, sodass eine automatische Positionskorrektur über die Behandlungstischfläche 3 möglich ist. Die drehbare Therapiequellen-Ultraschallsonde 13 kann dazu kontinuierlich oder sequentiell in zwei oder mehr bezüglich der Blickrichtung B gedrehten Ebenen Ultraschallbilder aufnehmen, damit auch Bewegungen des Zielobjekts T außerhalb der jeweiligen Sonographieebene getrackt werden können.

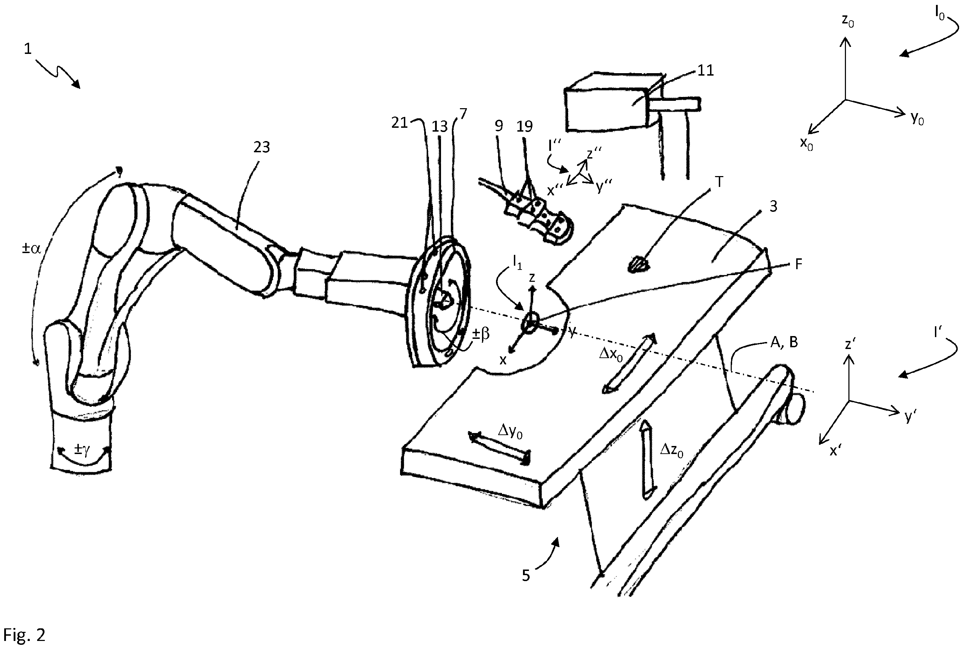

In dem in Fig. 2 gezeigten zweiten Ausführungsbeispiel ist im Gegensatz zum ersten Ausführungsbeispiel die Therapiequelle 7 nicht über Führungsbögen 15, 17 aufgehängt, sondern über einen Roboter- oder Gelenkarm 23. Mit dem Roboter- oder Gelenkarm 23 kann

die Position $\begin{pmatrix} x \\ y \\ z \end{pmatrix}$ und Ausrichtung $\begin{pmatrix} \alpha \\ \beta \\ \gamma \end{pmatrix}$ der Therapie-

quellen-Ultraschallsonde 13 im Bezugssystem $I_0$ des Behandlungsraums nahezu ohne Einschränkung beliebig eingestellt werden. In dem gezeigten Ausführungsbeispiel ist zwar die Behandlungstischfläche 3 im Bezugssystem $I_0$ des Behandlungsraums in den Achsen $x_0$, $y_0$ und $z_0$ verfahrbar, allerdings ist dies nicht zwingend erforderlich. Durch den Roboter- oder Gelenkarm 23 hat die Therapiequelle 7 genügend Bewegungsfreiheitsgrade, nämlich 5, um die Therapiequelle 7 im Bezugssystem $I_1$ der Behandlungstischfläche 3 zu positionieren, ohne dass das Positionieren des Bezugssystems $I_1$ der Behandlungstischfläche 3 im Bezugssystem $I_0$ des Behandlungsraums erforderlich wäre. Dies hat zwei Vorteile. Zum einen kann eine Nachführung der Position der Therapiequelle 7 über den Roboter- oder Gelenkarm 23 ggf. schneller sein, da die Gesamtmasse der Therapiequelle 7 geringer sein kann als die Masse der Behandlungstischfläche 3 samt Patient. Zum anderen wird der Patient nicht durch eine Nachführung der Position der Behandlungstischfläche 3 beschleunigt bzw. abgebremst, was wiederum dazu führen kann, dass sich das Zielobjekt T durch die Trägheit des Patienten gegenüber der Behandlungstischfläche 3 bewegt. Im Übrigen ist das zweite Ausführungsbeispiel analog zum ersten Ausführungsbeispiel, wobei die Therapiequellen-Ultraschallsonde 13 ebenfalls in der Therapiequelle 7 um den Winkel β drehbar integriert ist.

Alternativ zu einer drehbar integrierten Therapiequellen-Ultraschallsonde 13 könnte in beiden Ausführungsformen die Therapiequelle 7 selbst um den Winkel β drehbar sein. Außerdem muss in beiden Ausführungsformen nicht unbedingt die Therapiequellen-Ultraschallsonde 13 zentral in der Therapiequelle 7 integriert sein, sodass die Blickrichtung B mit der Therapieachse A übereinstimmt. Alternativ dazu könnten sich die Blickrichtung B und die Therapieachse A im Fokusbereich F in einem spitzen Winkel von höchstens 30° schneiden.

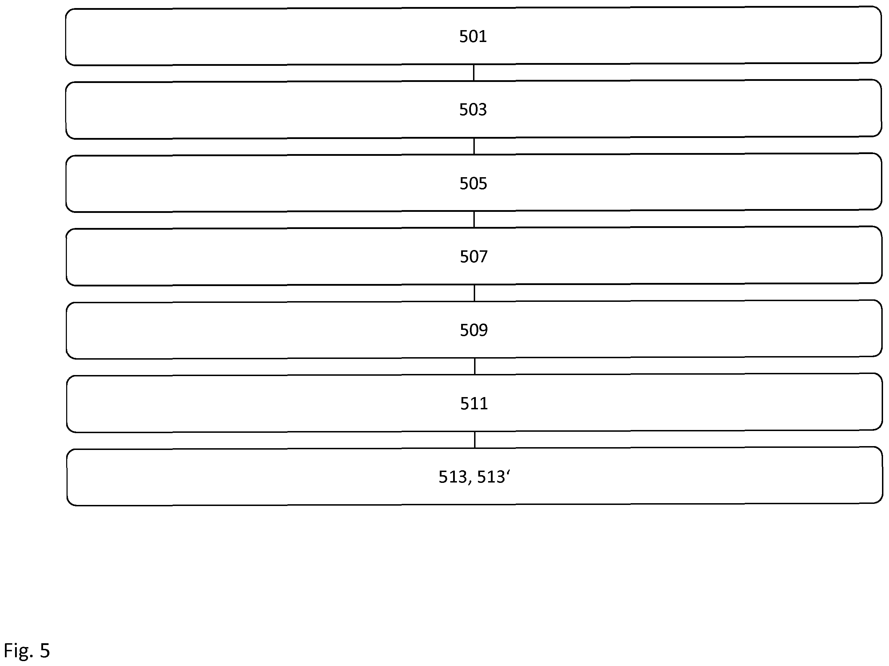

In Fig. 5 ist eine beispielhafte Ausführungsform des hierin offenbarten Steuerungsverfahrens für das oben beschriebene System gezeigt. In einem ersten Schritt wird dabei die räumliche Position und Ausrichtung der Freihand-Ultraschallsonde 9 im Bezugssystem der Behandlungstischfläche 3 mittels des Ortungssystems vorzugsweise kontinuierlich erfasst (501). In einem zweiten Schritt werden sechs die räumliche Position und Ausrichtung der Freihand-Ultraschallsonde 9 bestimmende Koordinaten gespeichert (503), vorzugsweise auf Befehl des Anwenders. Ein dritter Schritt umfasst ein automatisches oder manuelles Ausrichten (505) der Therapiequelle 7 basierend auf den gespeicherten sechs Koordinaten in den zwei Winkelebenen xz, yz im Bezugssystem der Behandlungstischfläche 3, sodass die an eine festgelegte Position im Bezugssystem der Therapiequelle 7 positionierte Therapiequellen-Ultraschallsonde 13 im Wesentlichen dieselbe Blickrichtung B hat wie die Freihand-Ultraschallsonde 9 zum Zeitpunkt des Speicherns (503) der sechs Koordinaten.

Außerdem wird in einem weiteren Schritt die an der festgelegten Position im Bezugssystem der Therapiequelle 7 positionierte Therapiequellen-Ultraschallsonde 13 um eine in ihrer Blickrichtung B verlaufende Drehachse y' im Bezugssystem der Therapiequelle 7 gedreht (507). Somit kann vor oder während eines Behandlungszyklus die Blickebene der Therapiequellen-Ultraschallsonde 13 gedreht werden, um in verschiedenen Blickebenen das Zielobjekt T tracken zu können, ohne die Therapiequelle 7 selbst drehen zu müssen.

Des Weiteren werden die zwei Winkelpositionen α, γ der Therapiequelle 7 bzw. der Therapiequellen-Ultraschallsonde 13 mittels des Ortungssystems 11 im Bezugssystem $I_1$ der Behandlungstischfläche 3 erfasst (509). Damit können während oder vor einem Behandlungszyklus die zwei Winkelpositionen α, γ der Therapiequelle 7 bzw. der Therapiequellen-Ultraschallsonde 13 mittels des Ortungssystems erfasst und entsprechend nachgeführt werden, um den Fokusbereich F im Bereich des Zielobjekts T zu halten.

Zusätzlich zu den zwei Winkelpositionen α, γ wird hier kontinuierlich die Position der Therapiequellen-Ultraschallsonde 13 innerhalb des Ortungsbereichs im Bezugssystem der Behandlungstischfläche mittels des Ortungssystems erfasst (511). Die Therapiequellen-Ultraschallsonde 13 wird außerdem im Bezugssystem der Behandlungstischfläche 3 mittels räumlichen Positionierens der Behandlungstischfläche 3 (s. Fig. 1) und/oder der Therapiequelle 7 (s. Fig. 2, beispielsweise über den Roboterarm 23) in den drei Achsen $x_0$, $y_0$ und $z_0$ um $\Delta x_0$, $\Delta y_0$ und $\Delta z_0$ basierend auf der kontinuierlichen Erfassung (511) positioniert (513).

Der Schritt des Positionierens (513) weist hier das Positionieren (513') eines im Bezugssystem der Therapiequelle 7 definierten Fokusbereichs F, basierend auf einer kontinuierlichen Erfassung der räumlichen Position der Therapiequellen-Ultraschallsonde 13 innerhalb des Ortungsbereichs im Bezugssystem $I_1$ der Behandlungstischfläche 3 und basierend auf einem Ultraschallbild der Therapiequellen-Ultraschallsonde 13 vom Zielobjekt T, mittels räumlichen Positionierens der Behandlungstischfläche 3 und/oder der Therapiequelle 7 in den drei Achsen $x_0$, $y_0$ und $z_0$ um $\Delta x_0$, $\Delta y_0$ und $\Delta z_0$ derart auf, dass der Fokusbereich F dem Zielobjekt T nachgeführt wird.

Die nummerierten Bezeichnungen der Bauteile, Bewegungsrichtungen oder Verfahrensschritte als "erste", "zweite", "dritte" usw. sind hierin rein willkürlich zur Unterscheidung der Bauteile, Bewegungsrichtungen oder Verfahrensschritte untereinander gewählt und können beliebig anders gewählt werden. Es ist damit kein Bedeutungs- oder Zeitrang verbunden, sofern dies nicht explizit beschrieben ist.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben. Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Im Übrigen soll hierin weder der Begriff "aufweisen" zusätzliche andere Merkmale oder Verfahrensschritte ausschließen noch soll "ein" oder "eine" eine Mehrzahl ausschließen.

**Patentansprüche**

1. System (1) zur medizinisch-therapeutischen Behandlung eines Zielobjekts (T) in einem auf einer Behandlungstischfläche (3) befindlichen Patienten, mit

- einer Freihand-Ultraschallsonde (9), die von einem Anwender des Systems (1) innerhalb eines Ortungsbereichs im Bezugssystem ($I_1$) der Behandlungstischfläche (3) manuell räumlich positionier- und ausrichtbar ist,
- einer Therapiequelle (7), die in mindestens zwei Winkelebenen im Bezugssystem der Behandlungstischfläche (3) auf das Zielobjekt (T) ausrichtbar ist,
- einer Therapiequellen-Ultraschallsonde (13), die an eine festgelegte Position im Bezugssystem (I') der Therapiequelle (7) räumlich positioniert oder positionierbar ist und deren Ausrichtung in dieser Position in den zwei Winkelebenen im Bezugssystem (I') der Therapiequelle (7) festgelegt ist,
- einem Ortungssystem (11) zur Erfassung der räumlichen Position und Ausrichtung der Freihand-Ultraschallsonde (9) innerhalb des Ortungsbereichs im Bezugssystem ($I_1$) der Behandlungstischfläche (3),
wobei das Ortungssystem (11) dazu ausgestaltet ist, sechs Koordinaten für eine vom Anwender gewählte räumliche Position und Ausrichtung der Freihand-Ultraschallsonde (9) mit einer Blickrichtung auf das Zielobjekt (T) zu speichern,
und wobei die Therapiequelle (7) dazu ausgestaltet ist, basierend auf den gespeicherten sechs Koordinaten in den mindestens zwei Winkelebenen so im Bezugssystem ($I_1$) der Behandlungstischfläche (3) auf das Zielobjekt (T) ausgerichtet zu werden, dass die Therapiequellen-Ultraschallsonde (13) im Bezugssystem ($I_1$) der Behandlungstischfläche (3) im Wesentlichen dieselbe Blickrichtung auf das Zielobjekt (T) hat wie die Freihand-Ultraschallsonde (9) zum Zeitpunkt des Speicherns der sechs Koordinaten.

2. System (1) nach Anspruch 1, wobei die an der festgelegten Position im Bezugssystem (I') der Therapiequelle (7) positionierte Therapiequellen-Ultraschallsonde (13) um eine in ihrer Blickrichtung (B) verlaufende Drehachse (y') im Bezugssystem (I') der Therapiequelle (7) drehbar ist.

3. System (1) nach Anspruch 1 oder 2, wobei die Blickrichtung (B) der an der festgelegten Position im Bezugssystem (I') der Therapiequelle (7) positionierten Therapiequellen-Ultraschallsonde (13) entlang der Schnittlinie der zwei Winkelebenen verläuft oder diese in einem Fokusbereich (F) in einem spitzen Schnittwinkel von höchstens 30° schneidet.

4. System (1) nach einem der vorhergehenden Ansprüche, wobei mindestens eine Winkelposition der an der festgelegten Position im Bezugssystem (I') der Therapiequelle (7) positionierten Therapiequellen-Ultraschallsonde (13) mittels des Ortungssystems (11) innerhalb des Ortungsbereichs im Bezugssystem ($I_1$) der Behandlungstischfläche (3) erfassbar ist.

5. System (1) nach einem der vorhergehenden Ansprüche, wobei die räumliche Position der Therapiequellen-Ultraschallsonde (13) innerhalb des Ortungsbereichs im Bezugssystem ($I_1$) der Behandlungstischfläche (3) mittels des Ortungssystems (11) kontinuierlich erfassbar ist, wobei die Therapiequellen-Ultraschallsonde (13) basierend auf dieser kontinuierlichen Erfassung im Bezugssystem ($I_1$) der Behandlungstischfläche (3) mittels räumlichen Positionierens der Behandlungstischfläche (3) und/oder der Therapiequelle (7) in drei Achsen positionierbar ist.

6. System (1) nach einem der vorhergehenden Ansprüche, wobei die Therapiequelle (7) einen Stoßwellengenerator eines extrakorporalen Stoßwellen-Lithotripsie-Systems (ESWL) aufweist.

7. System (1) nach einem der vorhergehenden Ansprüche, wobei das Ortungssystem dazu ausgestaltet ist, die sechs Koordinaten auf Befehl eines Anwenders zu speichern.

8. System (1) nach einem der vorhergehenden Ansprüche, wobei die Therapiequelle (7) einen definierten Fokusbereich (F) im Bezugssystem (I') der Therapiequelle (7) aufweist und die Therapiequelle (7) im Bezugssystem ($I_1$) der Behandlungstischfläche (3) basierend auf einer kontinuierlichen Erfassung der räumlichen Position der Therapiequellen-Ultraschallsonde (13) innerhalb des Ortungsbereichs im Bezugssystem ($I_1$) der Behandlungstischfläche (3) und basierend auf einem Ultraschallbild der Therapiequellen-Ultraschallsonde vom Zielobjekt (T) mittels räumlichen Positionierens der Behandlungstischfläche (3) und/oder der Therapiequelle (7) in drei Achsen derart positionierbar ist, dass der Fokusbereich (F) dem Zielobjekt (T) nachführbar ist.

9. Steuerungsverfahren für ein medizinisch-therapeutisches System (1) mit den Schritten:

   - Erfassen (501) der räumlichen Position und Ausrichtung einer Freihand-Ultraschallsonde (9) im Bezugssystem ($I_1$) einer Behandlungstischfläche (3) mittels eines Ortungssystems (11),
   - Speichern (503) von sechs die räumliche Position und Ausrichtung der Freihand-Ultraschallsonde (9) bestimmenden Koordinaten, und
   - Ausrichten (505) einer Therapiequelle (7) basierend auf den gespeicherten sechs Koordinaten in mindestens zwei Winkelebenen im Bezugssystem ($I_1$) der Behandlungstischfläche (3), sodass eine an eine festgelegte Position im

Bezugssystem (I') der Therapiequelle (7) positionierte Therapiequellen-Ultraschallsonde (13) im Wesentlichen dieselbe Blickrichtung (B) hat wie die Freihand-Ultraschallsonde (9) zum Zeitpunkt des Speicherns (503) der sechs Koordinaten.

10. Steuerungsverfahren nach Anspruch 9, mit dem Schritt:

- Drehen (507) der an der festgelegten Position im Bezugssystem (I') der Therapiequelle (7) positionierten Therapiequellen-Ultraschallsonde (13) um eine in ihrer Blickrichtung (B) verlaufende Drehachse (y') im Bezugssystem (I') der Therapiequelle (7).

11. Steuerungsverfahren nach Anspruch 9 oder 10, wobei die Blickrichtung (B) der an der festgelegten Position im Bezugssystem (I') der Therapiequelle (7) positionierten Therapiequellen-Ultraschallsonde (13) entlang der Schnittlinie der zwei Winkelebenen verläuft oder diese in einem Fokusbereich (F) in einem spitzen Schnittwinkel von höchstens 30° schneidet.

12. Steuerungsverfahren nach einem der Ansprüche 9 bis 11, mit dem Schritt:

- Erfassen (509) mindestens einer Winkelposition der an der festgelegten Position im Bezugssystem (I') der Therapiequelle (7) positionierten Therapiequellen-Ultraschallsonde (13) mittels des Ortungssystems (11) innerhalb des Ortungsbereichs im Bezugssystem ($I_1$) der Behandlungstischfläche (3).

13. Steuerungsverfahren nach einem der Ansprüche 9 bis 12, mit den Schritten:

- kontinuierlichem Erfassen (511) der räumlichen Position der Therapiequellen-Ultraschallsonde (13) innerhalb des Ortungsbereichs im Bezugssystem ($I_1$) der Behandlungstischfläche (3) mittels des Ortungssystems (11), und
- Positionieren (513) der Therapiequellen-Ultraschallsonde (13) im Bezugssystem ($I_1$) der Behandlungstischfläche (3) mittels räumlichen Positionierens der Behandlungstischfläche (3) und/oder der Therapiequelle (7) in drei Achsen basierend auf der kontinuierlichen Erfassung (511).

14. Steuerungsverfahren nach einem der Ansprüche 9 bis 13, wobei der Schritt des Speicherns (503) der sechs Koordinaten auf Befehl eines Anwenders erfolgt.

15. Steuerungsverfahren nach einem der Ansprüche 9 bis 14, mit dem Schritt des Positionierens (513') eines im Bezugssystem (I') der Therapiequelle (7) definierten Fokusbereichs (7), basierend auf einer kontinuierlichen Erfassung (511) der räumlichen Position der Therapiequellen-Ultraschallsonde (13) innerhalb des Ortungsbereichs im Bezugssystem ($I_1$) der Behandlungstischfläche (3) und basierend auf einem Ultraschallbild der Therapiequellen-Ultraschallsonde (13) von einem Zielobjekt (T), mittels räumlichen Positionierens der Behandlungstischfläche (3) und/oder der Therapiequelle (7) in drei Achsen derart, dass der Fokusbereich (F) dem Zielobjekt (T) nachgeführt wird.

**Claims**

1. A system (1) for the medical-therapeutic treatment of a target object (T) in a patient who is located on a treatment table surface (3), with

- a freehand ultrasonic probe (9) which is spatially positionable and alignable by the user of the system (1) in a manual manner within a locating region in the reference system ($I_1$) of the treatment table surface (3),
- a therapy source (7) which is alignable onto the target object (T) in at least two angle planes in the reference system of the treatment table surface (3),
- a therapy source ultrasonic probe (13) which is positioned or positionable onto a fixed position in the reference system (I') of the therapy source (7) and whose alignment in this position is fixed in the two angle planes in the reference system (I') of the therapy source (7),
- a locating system (11) for detecting the spatial position and alignment of the freehand ultrasonic probe (9) within the locating region in the reference system ($I_1$) of the treatment table surface (3),

wherein the locating system (11) is designed to store six coordinates for a spatial position and alignment of the freehand ultrasonic probe (9) with a viewing direction onto the target object (T), said position and alignment being selected by the user,

and wherein the therapy source (7) is designed to be aligned onto the target object (T) in the reference system ($I_1$) of the treatment table surface (3) on the basis of the stored six coordinates in the at least two angle planes, such that the therapy source ultrasonic probe (13) in the reference system ($I_1$) of the treatment table surface (3) essentially has the same viewing direction onto the target object (T) as the freehand ultrasonic probe (9) at the point in time of the storing of the six coordinates.

2. A system (1) according to claim 1, wherein the therapy source ultrasonic probe (13) which is positioned at the fixed position in the reference system (I') of the therapy source (7) is rotatable about a rotation axis (y') in the reference system (I') of the therapy source (7), said rotation axis running in the viewing direction (B) of said probe.

3. A system (1) according to claim 1 or 2, wherein the viewing direction (B) of the therapy source ultrasonic probe (13) which is positioned at the fixed position in the reference system (I') of the therapy source (7) runs along the section line of the two angle planes or intersects this in a focus region (F) at an acute intersection angle of at the most 30°.

4. A system (1) according to one of the preceding claims, wherein at least one angular position of the therapy source ultrasonic probe (13) which is positioned at the fixed position in the reference system (I') of the therapy source (7) can be detected within the locating region in the reference system ($I_1$) of the treatment table surface (3) by way of the locating system (11).

5. A system (1) according to one of the preceding claims, wherein the spatial position of the therapy source ultrasonic probe (13) within the locating region in the reference system ($I_1$) of the treatment table surface (3) can be continuously detected by way of the locating system (11), wherein the therapy source ultrasonic probe (13) can be positioned in three axes by way of the spatial positioning of the treatment table surface (3) and/or the therapy source (7), on the basis of this continuous detection in the reference system ($I_1$) of the treatment table surface (3).

6. A system (1) according to one of the preceding claims, wherein the therapy source (7) comprises a shock wave generator of an extra-corporal shock wave lithotripsy system (ESWL).

7. A system (1) according to one of the preceding claims, wherein the locating system is designed to store the six coordinates at the command of a user.

8. A system (1) according to one of the preceding claims, wherein the therapy source (7) comprises a defined focus region (F) in the reference system (I') of the therapy source (7), and the therapy source (7) in the reference system ($I_1$) of the treatment table surface (3) and on the basis of a continuous detection of the spatial position of the therapy source ultrasonic probe (13) within the locating region in the reference system ($I_1$) of the treatment table surface (3) and on the basis of an ultrasonic picture of the therapy source ultrasonic probe of the target object (T) is positionable in three axes by way of the spatial positioning of the treatment table surface (3) and/or of the therapy source (7), in a manner such that the focus region (F) can be tracked to the target object (T).

9. A control method for a medical-therapeutic system (1) with the steps:

   - detecting (501) the spatial position and alignment of a freehand ultrasonic probe (9) in the reference system ($I_1$) of a treatment table surface (3) by way of a locating system (11),
   - storing (503) six coordinates which determine the spatial position and alignment of the freehand ultrasonic probe (9), and
   - aligning (505) a therapy source (7) in at least two angle planes in the reference system ($I_1$) of the treatment table surface (3) on the basis of the stored six coordinates, so that a therapy source ultrasonic probe (13) which is positioned onto a fixed position in the reference system (I') of the therapy source (7) essentially has the same viewing direction (B) as the freehand ultrasonic probe (9) at the point in time of storing (503) the six coordinates.

10. A control method according to claim 9, with the step:

    - rotating (507) the therapy source ultrasonic probe (13) which is positioned at the fixed position in the reference system (I') of the therapy source (7), about a rotation axis (y') in the reference system (I') of the therapy source (7), said rotation axis running in the viewing direction (B) of said probe.

11. A control method according to claim 9 or 10, wherein the viewing direction (B) of the therapy source ultrasonic probe (13) which is positioned at the fixed position in the reference system (I') of the therapy source (7) runs along the section line of the two angle planes or intersects this in a focus region (F) at an acute intersection angle of at the most 30°.

12. A control method according to one of the claims 9 to 11, with the step:

    - detecting (509) at least one angle position of the therapy source ultrasonic probe (13) which is positioned at the fixed position in the reference system (1') of the therapy source (7), within the locating region in the reference system ($I_1$) of the treatment table surface (3) by way of the locating system (11).

**13.** A control method according to one of the claims 9 to 12, with the steps

- continuously detecting (511) the spatial position of the therapy source ultrasonic probe (13) within the locating region in the reference system ($I_1$) of the treatment table surface (3) by way of the locating system (11), and
- positioning (513) the therapy source ultrasonic probe (13) in the reference system ($I_1$) of the treatment table surface (3) in three axes on the basis of the continuous detection (511), by way of spatially positioning the treatment table surface (3) and/or the therapy source (7).

**14.** A control method according to one of the claims 9 to 13, wherein the step of storing (503) the six coordinates is effected at the command of the user.

**15.** A control method according to one of the claims 9 to 14, with the step of positioning (513') a focus region (7) which is defined in the reference system (I') of the therapy source (7), on the basis of a continuous detection (511) of the spatial position of the therapy source ultrasonic probe (13) within the locating region in the reference system ($I_1$) of the treatment table surface (3) and on the basis of an ultrasonic picture of the therapy source ultrasonic probe (13) of the target object (T), in three axes by way of the spatial positioning of the treatment table surface (3) and/or of the therapy source (7), in a manner such that the focus region (F) is tracked to the target object (T).

**Revendications**

**1.** Système (1) destiné au traitement médico-thérapeutique d'un objet cible (T) chez un patient se trouvant sur une surface de table de traitement (3), comprenant

- une sonde à ultrasons à main (9) qui peut être positionnée dans l'espace et orientée de manière manuelle par un utilisateur du système (1) dans une région de localisation au sein d'un système de référence ($I_1$) de la surface de table de traitement (3),
- une source thérapeutique (7) pouvant être orientée sur l'objet cible (T) selon au moins deux plans angulaires au sein du système de référence de la surface de table de traitement (3),
- une sonde à ultrasons de source thérapeutique (13) positionnée ou pouvant être positionnée dans l'espace au niveau d'une position spécifiée au sein d'un système de référence (I') de la source thérapeutique (7) et dont l'orientation dans ladite position est spécifiée selon les deux plans angulaires au sein du système de référence (I') de la source thérapeutique (7),
- un système de localisation (11) destiné à détecter la position spatiale et l'orientation de la sonde à ultrasons à main (9) dans la région de localisation au sein du système de référence ($I_1$) de la surface de table de traitement (3),

dans lequel le système de localisation (11) est conçu pour mémoriser six coordonnées pour une position spatiale et une orientation, sélectionnées par l'utilisateur, de la sonde à ultrasons à main (9) avec une direction de visée sur l'objet cible (T),

et dans lequel la source thérapeutique (7) est conçue pour être orientée sur l'objet cible (T) au sein du système de référence ($I_1$) de la surface de table de traitement (3), en se basant sur les six coordonnées mémorisées selon les plans angulaires, au moins au nombre de deux, de sorte que la sonde à ultrasons de source thérapeutique (13) présente essentiellement la même direction de visée sur l'objet cible (T) au sein du système de référence ($I_1$) de la surface de table de traitement (3) que la sonde à ultrasons à main (9) au moment de la mémorisation des six coordonnées.

**2.** Système (1) selon la revendication 1, dans lequel la sonde à ultrasons de source thérapeutique (13) positionnée au niveau de la position spécifiée au sein du système de référence (I') de la source thérapeutique (7) peut tourner au sein du système de référence (I') de la source thérapeutique (7) autour d'un axe de rotation (y') passant par la direction de visée (B) de ladite sonde.

**3.** Système (1) selon la revendication 1 ou 2, dans lequel la direction de visée (B) de la sonde à ultrasons de source thérapeutique (13) positionnée au niveau de la position spécifiée au sein du système de référence (I') de la source thérapeutique (7) s'étend le long de la ligne d'intersection des deux plans angulaires ou coupe ladite ligne dans une région focale (F) avec un angle d'intersection aigu inférieur ou égal à 30°.

**4.** Système (1) selon l'une des revendications précédentes, dans lequel au moins une position angulaire de la sonde à ultrasons de source thérapeutique (13) positionnée au niveau de la position spécifiée au sein du système de référence (I') de la source thérapeutique (7) peut être détectée au moyen du système de localisation (11) dans la région de localisation au sein du système de référence ($I_1$) de la surface de table de traitement (3).

**5.** Système (1) selon l'une des revendications précédentes, dans lequel la position spatiale de la sonde à ultrasons de source thérapeutique (13) peut être détectée de manière continue dans la région de localisation au sein du système de référence (I$_1$) de la surface de table de traitement (3) au moyen du système de localisation (11), dans lequel la sonde à ultrasons de source thérapeutique (13) peut être positionnée sur trois axes en se basant sur ladite détection en continu au sein du système de référence (I$_1$) de la surface de table de traitement (3) au moyen d'un positionnement spatial de la surface de table de traitement (3) et/ou de la source thérapeutique (7).

6. Système (1) selon l'une des revendications précédentes, dans lequel la source thérapeutique (7) présente un générateur d'ondes de choc d'un système de lithotripsie extracorporelle par ondes de choc (LEOC).

7. Système (1) selon l'une des revendications précédentes, dans lequel le système de localisation est conçu pour mémoriser les six coordonnées sur ordre d'un utilisateur.

8. Système (1) selon l'une des revendications précédentes, dans lequel la source thérapeutique (7) présente au sein du système de référence (I') de la source thérapeutique (7) une région focale (F) définie, et la source thérapeutique (7) peut être positionnée sur trois axes au sein du système de référence (I$_1$) de la surface de table de traitement (3), en se basant sur une détection continue de la position spatiale de la sonde à ultrasons de source thérapeutique (13) dans la région de localisation au sein du système de référence (I$_1$) de la surface de table de traitement (3), et en se basant sur une image échographique de l'objet cible (T) fournie par la sonde à ultrasons de source thérapeutique (7), au moyen d'un positionnement spatial de la surface de table de traitement (3) et/ou de la source thérapeutique (7) de telle manière que la région focale (F) peut être asservie à l'objet cible (T).

9. Procédé de commande d'un système médico-thérapeutique (1) comprenant les étapes consistant à :

    - détecter (501) la position spatiale et l'orientation d'une sonde à ultrasons à main (9) au sein du système de référence (I$_1$) d'une surface de table de traitement (3) au moyen d'un système de localisation (11),
    - mémoriser (503) six coordonnées déterminant la position spatiale et l'orientation de la sonde à ultrasons à main (9), et
    - orienter (505) une source thérapeutique (7), en se basant sur les six coordonnées mémorisées, selon au moins deux plans angulaires au sein du système de référence (I$_1$) de la surface

de table de traitement (3), de sorte qu'une sonde à ultrasons de source thérapeutique (13) positionnée au niveau d'une position spécifiée au sein du système de référence (I') de la source thérapeutique (7) présente essentiellement la même direction de visée (B) que la sonde à ultrasons à main (9) au moment de la mémorisation (503) des six coordonnées.

10. Procédé de commande selon la revendication 9, comprenant l'étape consistant à :

    - faire pivoter (507) la sonde à ultrasons de source thérapeutique (13), positionnée au niveau de la position spécifiée au sein du système de référence (11) de la source thérapeutique (7), autour d'un axe de rotation (y') passant par la direction de visée (B) de ladite sonde au sein du système de référence (I') de la source thérapeutique (7).

11. Procédé de commande selon la revendication 9 ou 10, dans lequel la direction de visée (B) de la sonde à ultrasons de source thérapeutique (13) positionnée au niveau de la position spécifiée au sein du système de référence (I') de la source thérapeutique (7) s'étend le long de la ligne d'intersection des deux plans angulaires ou coupe ladite ligne dans une région focale (F) avec un angle d'intersection aigu inférieur ou égal à 30°.

12. Procédé de commande selon l'une des revendications 9 à 11, comprenant l'étape consistant à :

    - détecter (509) au moins une position angulaire de la sonde à ultrasons de source thérapeutique (13) positionnée au niveau de la position spécifiée au sein du système de référence (I') de la source thérapeutique (7) au moyen du système de localisation (11) dans la région de localisation au sein du système de référence (I$_1$) de la surface de table de traitement (3).

13. Procédé de commande selon l'une des revendications 9 à 12, comprenant les étapes consistant à :

    - détecter de manière continue (511) la position spatiale de la sonde à ultrasons de source thérapeutique (13) dans la région de localisation au sein du système de référence (I$_1$) de la surface de table de traitement (3) au moyen du système de localisation (11), et
    - positionner (513) la sonde à ultrasons de source thérapeutique (13) au sein du système de référence (I$_1$) de la surface de table de traitement (3) au moyen d'un positionnement spatial de la surface de table de traitement (3) et/ou de la source thérapeutique (7) sur trois axes en se basant sur la détection continue (511).

**14.** Procédé de commande selon l'une des revendications 9 à 13, dans lequel l'étape de mémorisation (503) des six coordonnées est mise en œuvre sur ordre d'un utilisateur.

**15.** Procédé de commande selon l'une des revendications 9 à 14, comprenant l'étape de positionnement (513') d'une région focale (7) définie au sein du système de référence (I') de la source thérapeutique (7), en se basant sur une détection continue (511) de la position spatiale de la sonde à ultrasons de source thérapeutique (13) dans la région de localisation au sein du système de référence ($I_1$) de la surface de table de traitement (3) et en se basant sur une image échographique d'un objet cible (T) fournie par la sonde à ultrasons de source thérapeutique (13), au moyen d'un positionnement spatial de la surface de table de traitement (3) et/ou de la source thérapeutique (7) sur trois axes de telle manière que la région focale (F) est asservie à l'objet cible (T).

Fig. 1

Fig. 2

EP 3 453 343 B1

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010055836 B4

- EP 2340781 B1